# EUROPÄISCHE PATENTSCHRIFT

(11) **EP 1 250 842 B1**
(45) Veröffentlichungstag und Bekanntmachung des Hinweises auf die Patenterteilung: **17.12.2003**
(21) Anmeldenummer: 02007784.8
(22) Anmeldetag: 06.04.2002
(51) Int. Cl.: A01N 37/12, A61K 7/50, A61K 7/32

(54) **Mittel zur Bekämpfung von Mikroorganismen, enthaltend einen wirksamen Gehalt an Estern des Polyglycerins**
Composition for controlling microorganisms containing an effective amount of polyglycerinesters
Composition pour combattre les microorganismes contenant une quantité efficace d'esters de polyglycerine

(30) Priorität: 20.04.2001 DE 10119694
(43) Veröffentlichungstag der Anmeldung: 23.10.2002
(73) Patentinhaber: Goldschmidt AG, 45127 Essen (DE)
(72) Erfinder: Brock, Achim, Dr., 45326 Essen (DE); Grüning, Burghard, Dr., 45134 Essen (DE); Hills, Geoffrey, Dr., 45355 Essen (DE)

(56) Entgegenhaltungen:
- EP-A- 0 344 418
- EP-A- 0 407 959
- EP-A- 0 484 529
- EP-A- 0 895 808
- WO-A-94/09753
- WO-A-94/18943
- WO-A-95/23586
- PATENT ABSTRACTS OF JAPAN vol. 1999, no. 05, 31. Mai 1999 (1999-05-31) & JP 11 029413 A (MEIJI MILK PROD CO LTD), 2. Februar 1999 (1999-02-02)
- PATENT ABSTRACTS OF JAPAN vol. 1998, no. 14, 31. Dezember 1998 (1998-12-31) & JP 10 234294 A (LION CORP), 8. September 1998 (1998-09-08)
- PATENT ABSTRACTS OF JAPAN vol. 1998, no. 13, 30. November 1998 (1998-11-30) & JP 10 225282 A (DAI ICHI KOGYO SEIYAKU CO LTD), 25. August 1998 (1998-08-25)
- PATENT ABSTRACTS OF JAPAN vol. 011, no. 115 (C-415), 10. April 1987 (1987-04-10) & JP 61 257191 A (MEITO SANGYO KK), 14. November 1986 (1986-11-14)

## Beschreibung

Gegenstand der Erfindung sind kosmetische Verfahren zur Bekämpfung von Mikroorganismen, enthaltend einen wirksamen Gehalt an enzymatisch hergestellten Estern des Polyglycerins.

Zur Bekämpfung von Mikroorganismen (grampositive Bakterien, gramnegative Bakterien, Mykobakterien, Dermatophyten, Sprossund Fadenpilze, Viren und Sporen) welche an der Oberfläche von Haut und Haaren, Kleidung, Geräten der Körperreinigung und - pflege wie beispielsweise im Dentalbereich, medizinischen Instrumenten aber auch Räumen und Einrichtungsgegenständen vorhanden sind, sind eine Vielzahl antimikrobiell wirksamer chemischer Substanzen bzw. Gemische dieser Substanzen bekannt, welche nach ihrem Verwendungszweck in Desinfektionsmittel, Konservierungsmittel, Antiseptika und kosmetische Wirkstoffe, um einige aufzuzählen, eingeteilt werden.

Die wesentlichen Vertreter dieser Gruppen sind: Aldehyde wie Formaldehyd, Glyoxal oder Glutaraldehyd; Phenol-Derivate wie 2.2'-Dihydroxybiphenyl und 4-Chlor-3-methylphenol; quaternäre Ammoniumverbindungen, Kationentenside wie Benzalkoniumchlorid, Cetrimoniumbromid, Cetylpyridiniumchlorid; Amphotenside sowie aktiven Sauerstoff abspaltende Verbindungen wie beispielsweise Wasserstoffperoxid, organische Persäuren, Alkylperoxide oder Alkylhydroperoxide.

Diese weisen allerdings etliche Nachteile auf, da sie die vielfältigen Forderungen, die in der Praxis an sie gestellt werden, wie z. B. breites Wirkungsspektrum, kurze Einwirkungszeiten bei niedrigen Temperaturen, gute Hautverträglichkeit, geringe Toxizität, Materialverträglichkeit, nicht oder nur unzureichend erfüllen.

Desinfektionsmittel auf Aldehyd- oder Phenolbasis gelten als toxikologisch und ökologisch bedenklich, führen häufig zu Sensibilisierungen insbesondere der Haut und Atmungsorgane und weisen darüber hinaus einen charakteristischen, durchdringenden und unangenehmen Geruch auf. Einige gelten zudem als potentielle Canzerogene.

Quaternäre Ammoniumverbindungen (Quats) sind toxikologisch weitestgehend unbedenklich, weisen keine oder nur sehr geringere Hautsensibilisierung auf und sind praktisch geruchlos. Sie besitzen jedoch eine beträchtliche hautreizende Wirkung. Bei Einsatz von Quats können sich wie bei der Verwendung von Aldehyden unerwünschte Ablagerungen und Schichten auf den behandelten Flächen bilden, die optisch nachteilig in Erscheinung treten und sich durch übliche Reinigungsverfahren nur schwer oder überhaupt nicht wieder entfernen lassen.

Gemäß der JP-A-11-29413 werden Mischungen aus Polyglycerinmonofettsäureestern und Palyglycerindifettsäureestern sowie Sorbitolmonofettsäureestern als Biozide im Pflanzenschutz verwendet. Sie sollen excellente Eigenschaften für die Sprühanwendung aufeisen und für Menschen unbedenklich sein.

Aus der DE-A-42 37 081 sind kosmetische Deodorantien bekannt, welche als Wirkstoffe auf chemischem Wege hergestellte Fettsäureester des Di- und Triglycerins enthalten. Nach der dort gegebenen Lehre sind nur die Monoester wirksam zur Bekämpfung grampositiver Bakterien.

Diese Monoester können gemäß den bekannten chemischen Verfahren des Standes der Technik (DE-A-38 18 293) hergestellt werden durch alkalisch katalysierte Umsetzung eines 1,5 bis 2,5-fachen molaren Überschusses an Fettsäuren oder Fettsäurederivaten mit Isopropylidenderivaten des Di- und Triglycerins, nachfolgender Reinigung des Reaktionsproduktes und anschließender saurer Hydrolyse oder Alkoholyse der Isopropylidengruppen. Nach Beendigung der Reaktion muss die Lösung neutralisiert und die Monoester müssen isoliert und gereinigt werden. Neben der Vielstufigkeit der Synthese ist im Falle der Diglycerin-Derivate zusätzlich die Verwendung von equimolaren Mengen an Epichlorhydrin als Nachteil dieser Route zu bewerten.

Daneben sind auch enzymatisch katalysierte Verfahren zur Herstellung von Polyglycerinfettsäureestern bekannt. D. Charlemagne und M. D. Legoy (JAOCS 1995, Vol. 72, no. 1, 61-65) adsorbieren hierzu erst das Polyglycerin auf der gleichen Menge Kieselgel, bevor sie in Suspension mit Fettsäuremethylestern unter Lipasekatalyse reagieren lassen. Nachteilig ist hierbei vor allem der Verlust des teuren Enzyms, das nach Beendigung der Reaktion zusammen mit dem Kieselgel durch Filtration abgetrennt wird. S. Matsumura, M. Maki, K. Toshima und K. Kawada (J. Jpn. Oil Chem. Soc. 1999, Vol. 48, No. 7, 681-692) nutzen dieses Verfahren in Abwandlung, um unter Einsatz von 20 Gew.-% Enzym Polyglycerinester zu synthetisieren. Gemäß der in der DE-A-42 37 081 vermittelten Lehre reinigen sie unter hohem Aufwand mittels Säulenchromatographie weiter auf, um zu reinen Monoestern mit den bekannten antimikrobiellen Aktivitäten zu gelangen.

Aufgabe der Erfindung war es daher, kosmetische Verfahren zur Bekämpfung von Mikroorganismen zu finden, die den geschilderten Nachteilen der Mittel des Standes der Technik weitgehend Abhilfe schaffen, eine hohe antimikrobielle Wirkung zeigen und aus einfach zugänglichen Rohstoffen nach einem ökonomisch tragbaren und ökologisch unbedenklichen Verfahren unkompliziert hergestellt werden können.

Es war überraschend und für den Fachmann aufgrund der Lehren des Standes der Technik nicht vorauszusehen, dass Mischungen von Fettsäuremono-, -di- und -triestern des Polyglycerins, welche durch enzymatisch katalysierte Umsetzung hergestellt wurden, vergleichbare und teilweise sogar deutlich bessere Wirksamkeiten bei der Bekämpfung von Mikroorganismen aufweisen, als die durch chemische Synthese oder enzymatische Herstellung und Aufreinigung hergestellten Monoester.

Ein Gegenstand der Erfindung ist daher ein Verfahren zur Bekämpfung von grampositiven Bakterien, gramnegativen Bakterien, Mykobakterien, Dermatophyten, Spross- und Fadenpilzen, Viren und Sporen in kosmetischen Formulierungen unter Verwendung von Mono- und Diestern des Di- und/oder Triglycerins mit einem Verhältnis von Mono- zu Diestern im Bereich von 20:80 bis 80:20; erhätlich durch Umsetzung des Di- und/oder Triglycerins in Gegenwart von Lipasen und Esterasen als Ver-/Umesterungskatalysatoren mit geradkettigen oder verzweigten, gegebenenfalls OH-Gruppen und/oder Doppelbindungen enthaltenden Fettsäuren mit 6 bis 14 C-Atomen in der Hauptkette.

Ein weiterer Gegenstand der Erfindung ist ein Verfahren zur Bekämpfung von grampositiven Bakterien, gramnegativen Bakterien, Mykobakterien, Dermatophyten, Spross- und Fadenpilzen, Viren und Sporen in kosmetischen Formulierungen für den Bereich der Körperreinigung und Körperpflege.

Ein weiterer Gegenstand der Erfindung ist ein Verfahren zur Bekämpfung von grampositiven Bakterien, gramnegativen Bakterien, Mykobakterien, Dermatophyten, Spross- und Fadenpilzen, Viren und Sporen in kosmetischen Formulierungen gemäß Anspruch 1 zur Herstellung kosmetischer Formulierungen gegen Körpergeruch und gegen Schuppenbildung.

Ein weiterer Gegenstand der Erfindung ist ein Verfahren zur Bekämpfung von grampositiven Bakterien, gramnegativen Bakterien, Mykobakterien, Dermatophyten, Spross- und Fadenpilzen, Viren und Sporen in kosmetischen Formulierungen gemäß Anspruch 1 zur Herstellung von kosmetischen Formulierungen gegen unreine Haut und leichte Formen der Akne.

Die erfindungsgemäß verwendeten Polyglycerine sind zum einen lineare Verbindungen der allgemeinen Formel

HO-CH₂-CH(OH)-CH₂-O-[CH₂-CH(OH)-CH₂-O]ₙ-H

worin n = 1-9, vorzugsweise 1-6, insbesondere 1-3, speziell 1 und 2 bedeutet. Darüber hinaus können die verwendeten Polyglycerine auch verzweigt sein und cyclische Anteile enthalten.

Es sind bei Raumtemperatur hochviskose Flüssigkeiten, welche neben Diglycerin vor allem die höher kondensierten Oligomeren des Glycerins enthalten. Besonders bevorzugt im Sinne der vorliegenden Erfindung werden technische Gemische von Polyglycerinen eingesetzt, die üblicherweise Diglycerin, Triglycerin, Tetraglycerin und Pentaglycerin enthalten.

Sie können beispielsweise industriell hergestellt werden durch basenkatalytische Kondensation von Glycerin oder auch durch Hydrolyse und Kondensation von Epichlorhydrin. Darüber hinaus sind Polyglycerine auch zugänglich durch Polymerisation von Glycidol. Die Trennung und Isolation der einzelnen Polyglycerine ist durch Behandlung mit den verschiedenen im Stand der Technik bekannten Mitteln möglich. Eine Übersicht von G. Jakobson über die verschiedenen Syntheserouten ist zu finden in "Fette Seifen Anstrichmittel", 1986, 88. Jahrgang, Nr. 3, 101-106. Die verschiedenen Strukturmöglichkeiten für Polyglycerin können nachgelesen werden bei H. Dolhaine, W. Preuß und K. Wollmann (Fette Seifen Anstrichmittel 1984, 86. Jahrgang, Nr. 9, 339-343).

Handelsübliche Produkte sind im Allgemeinen Gemische von Polyglycerinen mit verschiedenen Kondensationsgraden, der maximale Kondensationsgrad kann darin in der Regel bis zu 10 in Ausnahmen auch größer sein. Sie enthalten etwa 0 bis 5 Gew-% Glycerin, 15 bis 40 Gew-% Diglycerin, 30 bis 55 Gew-% Triglycerin, 10 bis 25 Gew-% Tetraglycerin, 0 bis 10 Gew-% höhere Oligomere.
Die erfindungsgemäß bevorzugt verwendeten Polyglycerine enthalten 15 bis 35 Gew-% Diglycerin, 38 bis 52 Gew-% Triglycerin, 15 bis 25 Gew-% Tetraglycerin, < 10 Gew-% höhere Oligomere und < 2 Gew-% cyclische Verbindungen. Besonders bevorzugt werden Polyglycerine, die nur oder überwiegend Diglycerin enthalten, verwendet.

Die im Sinne der vorliegenden Erfindung vorzugsweise einzusetzenden Fettsäuren und Fettsäurederivate sowie deren Gemische leiten sich von gradkettigen oder verzweigten, gesättigten, einfach oder mehrfach ungesättigten Carbon- und Fettsäureresten mit 6 bis 14 C-Atomen, vorzugsweise 8 bis 12, insbesondere 8 bis 10 C-Atomen in der Hauptkette ab.

Als Fettsäurederivate können alle üblichen Derivate eingesetzt werden, die Ver-/Umesterungsreaktionen eingehen. Erfindungsgemäß sind die Fettsäurederivate besonders bevorzugt ausgewählt aus Fettsäurealkylestern mit 1 bis 4 C-Atomen im Alkoholrest.

Als Fettsäuren oder deren Ester werden einzeln oder in Mischungen Fettsäuren wie Capronsäure, Caprylsäure, Caprinsäure, 2-Ethylhexansäure, Undecylensäure, Laurinsäure und Myristinsäure verwendet. Geeignet sind prinzipiell alle Fettsäuren mit ähnlicher Kettenverteilung.

Vorzugsweise werden Caprylsäure und Caprinsäure verwendet.

Das Stoffmengenverhältnis von Fettsäure oder Fettsäurederivaten zu Polyglycerin wird so eingestellt, dass im Reaktionsgemisch ein Überschuss an Hydroxylgruppen gegenüber Fettsäureresten besteht. Bevorzugt im Sinne der vorliegenden Erfindung ist es, das Stoffmengenverhältnis von Mol Fettsäurederivaten zu Mol Polyglycerin im Verhältnis von 0,25:1 bis 4:1, insbesondere 0,5:1 bis 2:1 einzustellen.

Die enzymatische Ver- und Umesterung mittels Enzymen, insbesondere immobilisierter Enzyme, wird vorzugsweise mit solchen Enzymen ausgeführt, die ausgewählt sind aus der Gruppe der Lipasen, Esterasen oder Proteasen, insbesondere Lipasen. Sie besitzen Enzymkatalyseaktivität für Esterbindungen, insbesondere zur Hydrolyse, Ver- und Umesterung. Derartige Lipasen sind in der WO 90/09451 beschrieben. Darüber hinaus ist das Produkt Novozym® 435 der Firma Novozymes als immobilisiertes Lipasesystem bekannt und im Handel erhältlich. Dieses Enzym wird besonders bevorzugt im Sinne der vorliegenden Erfindung eingesetzt.

Die erfindungsgemäßen Polyglycerinfettsäureester bestehen zusammenfassend aus einem Gemisch von Verbindungen unterschiedlichen Veresterungsgrades, das beträchtliche Anteile nicht veresterten Polyglycerins enthalten kann. Das zu Grunde liegende Polyglycerin kann dabei einheitlich oder seinerseits wiederum ein Gemisch von Produkten unterschiedlichen Kondensationsgrades sein.

In dem erfindungsgemäßen Verfahren zur Bekämpfung von Mikroorganismen können je nach Einsatzzweck darüber hinaus noch auf diesem Gebiet übliche anionische, nichtionische, kationische und/oder amphotere Tenside mitverwendet werden.

Typische Beispiele für solche Tenside sind:
1. Nichtionische Tenside basierend auf Alkylenoxiden wie Ethoxylate langkettiger verzweigter Alkohole, Ethoxylate von Sorbitanestern, Propylenoxidethylenoxidcopolymere, Hydroxyalkylfettsäureamide, Polydimethylsiloxanpolyalkylenoxidcopolymere, Zucker basierte Tenside wie Alkylpolyglycoside, Alkylglycosidester, N-Acylglucamide und Polyglycerinester,
2. Anionische Tenside wie Alkyl- und Alkylethersulfate, α-Olefinsulfonate, Fettsäureestersulfonate, Alkylarylsulfonate, Sulfosuccinate, Phosphorsäurealkyl- oder alkoxyalkylester, Taurate, N-Acylaminosäurederivate, Sarcosinate, Isethionate und Seifen,
3. Kationische Tenside wie Alkyltrimethylammoniumsalze, Fettsäureester von Di- oder Triethanolammoniumsalzen, Alkylimidazoliniumsalzen, Acylamidopropyldimethylammoniumsalze, kationisch derivatisierte Polydimethylsiloxane,
4. Zwitterionische und amphotere Tenside wie Betaine, Sulfobetaine, Aminoxide und Amphoacetate.

Kosmetische Verfahren auf Basis der beschriebenen enzymatisch hergestellten Polyglycerinfettsäureester werden insbesondere eingesetzt zur Bekämpfung von Körpergeruch, von Schuppen oder zur Bekämpfung von Unreinheiten der Haut. Sie können als solche in Form homogener Flüssigkeiten, als Gele, als Salben, als wachsartige oder emulsionsartige Zubereitungen formuliert sein. Insbesondere in der Emulsionsform werden sie Öle wie Esteröle, flüchtige oder weniger flüchtige Siliconderivate wie Decamethylcyclopentasiloxan, Paraffinöle und dergleichen enthalten.

Es kann von Vorteil sein, in den erfindungsgemäßen Verfahren zur Bekämpfung von Mikroorganismen andere antimikrobiell wirksame Stoffe mitzuverwenden. Als solche seien genannt Triclosan, Farnesol, Glycerinmonolaurat oder 2-Ethylhexyloxyglycerin. Sie können je nach Anwendungszweck neben den genannten Tensiden noch die jeweils spezifischen Hilfs- und Zusatzstoffe beispielsweise Lösungsmittel, Gerüststoffe, Schauminhibitoren, Salze, Bleichmittel, Bleichaktivatoren, optische Aufheller, Vergrauungsinhibitoren, Lösungsvermittler, Verdickungsmittel, Duftund Farbstoffe, Emulgatoren, biogene Wirkstoffe wie Pflanzenextrakte und Vitaminkomplexe enthalten. Als Lösungsmittel kommen insbesondere in Frage Wasser oder Alkohole wie beispielsweise Ethanol, Propanol, Isopropanol, 2-Methyl-2-propanol, Propylenglykol, Dipropylenglykol oder Glycerin.

Die jeweils einzusetzenden Mengen an derartigen Zusatzstoffen sind in Abhängigkeit von der Art des jeweiligen Produktes dem Fachmann bekannt oder können im Bedarfsfall durch einfaches Ausprobieren leicht ermittelt werden.

Weitere Verwendungsmöglichkeiten für die erfindungsgemäßen Mittel ist deren Einsatz als Konservierungsmittel in Lebensmitteln und in Lebensmittelverpackungen, in denen sie in der Regel in Konzentrationen von 0,01 bis 5 Gew.-%, bevorzugt 0,1 bis 1 Gew.-% eingesetzt werden. Lebensmitteln können die erfindungsgemäßen Ester einfach in entsprechender Menge zugesetzt werden. Zur Anwendung in Verpackungen kommen die Polyglycerinester, indem beispielsweise Papiere mit einer Lösung oder Emulsion der Ester getränkt werden oder Folien mit entsprechenden Zubereitungen der Ester besprüht werden. Die Ester können auch vor oder während des Formgebungsprozesses der Verpackungen wie der Extrusion zugesetzt werden.

### Beispiel 1: Diglycerincaprinat

In einem Dreihalskolben mit KPG-Rührer und aufgesetzter Destillationsbrücke werden 415 g Diglycerin und 431 g Caprinsäure eingewogen und bei 60 °C mit 16,9 g Novozym® 435 versetzt. Das entstehende Reaktionswasser wird im Wasserstrahlvakuum entfernt, bis die Säurezahl der Reaktionsmischung auf einen Wert kleiner 2 gefallen ist. Zur Abtrennung des Enzyms wird das Produkt abschließend filtriert.

### Beispiel 2: Polyglycerin-3-caprinat

In einem Dreihalskolben mit KPG-Rührer und aufgesetzter Destillationsbrücke werden 460 g eines Polyglycerins gekennzeichnet durch folgende Verteilung (Gew.-%): 0,2 Glycerin, 32,6 Diglycerin, 41,2 Triglycerin, 14,8 Tetraglycerin, 3,9 Pentaglycerin, 1,9 Hexaglycerin, 5,4 höhere Polyglycerine und 345 g Caprinsäure eingewogen und bei 60 °C mit 16,1 g Novozym® 435 versetzt. Das entstehende Reaktionswasser wird im Wasserstrahlvakuum entfernt, bis die Säurezahl der Reaktionsmischung auf einen Wert kleiner 2 gefallen ist. Zur Abtrennung des Enzyms wird das Produkt abschließend filtriert.

### Beispiel 3: Diglycerincaprylat

In einem Dreihalskolben mit KPG-Rührer und aufgesetzter Destillationsbrücke werden 415 g Diglycerin und 361 g Caprylsäure eingewogen und bei 60 °C mit 15,5 g Novozym® 435 versetzt. Das entstehende Reaktionswasser wird im Wasserstrahlvakuum entfernt, bis die Säurezahl der Reaktionsmischung auf einen Wert kleiner 2 gefallen ist. Zur Abtrennung des Enzyms wird das Produkt abschließend filtriert.

### Beispiel 4: Polyglycerin-3-caprylat

In einem Dreihalskolben mit KPG-Rührer und aufgesetzter Destillationsbrücke werden 579 g eines Polyglycerins gekennzeichnet durch folgende Verteilung (Gew.-%): 0,2 Glycerin, 32,6 Diglycerin, 41,2 Triglycerin, 14,8 Tetraglycerin, 3,9 Pentaglycerin, 1,9 Hexaglycerin, 5,4 höhere Polyglycerine und 363 g Caprylsäure eingewogen und bei 60 °C mit 18,8 g Novozym® 435 versetzt. Das entstehende Reaktionswasser wird im Wasserstrahlvakuum entfernt, bis die Säurezahl der Reaktionsmischung auf einen Wert kleiner 2 gefallen ist. Zur Abtrennung des Enzyms wird das Produkt abschließend filtriert.

### Beispiel 5: Mikrobiologische Tests

Die Wirksamkeit der erfindungsgemäßen Produkte wird mit Hilfe der "Prüfung auf ausreichende Konservierung" (gemäß der Europäischen Pharmaverordnung) festgestellt. Hierbei zeigt sich, dass die erfindungsgemäßen Produkte gegenüber dem Stand der Technik weit überlegen sind.

### Durchführung der mikrobiologischen Tests:

### A) Gegen Corynebacterium xerosis, Staphylococcus epidermidis und Candida albicans

### 1. Proben und Material

1.1. Proben
   a. Diglycerinmonocaprinat (D-Caprate A, Solvay Alkali GmbH; Vergleichssubstanz gemäß dem Stand der Technik)
   b. Diglycerincaprinat (Ausführungsbeispiel 1)
   c. Polyglycerin-3-caprinat (Ausführungsbeispiel 2)
   d. Diglycerincaprylat (Ausführungsbeispiel 3)
   e. Polyglycerin-3-caprylat (Ausführungsbeispiel 4)
1.2. Testkeime
   Corynebacterium xerosis DSM 20743
   Staphylococcus epidermidis DSM 3269
   Candida albicans ATCC 10231
1.3. Verwendete Medien
   Nährmedien:
   CSL: Caseinpepton-Sojamehlpepton-Lösung
   CSA: Caseinpepton-Sojamehlpepton-Agar
   Sabouraud-Glucose-Bouillon/Agar
   Verdünnungsflüssigkeit mit Inaktivierungszusätzen
   NaCl-Pepton-Pufferlösung mit EG-Enthemmer (3 % Tween® 80, 0,3 % Lecithin, 0,1 % Histidin, 0,5 % Na-Thiosulfat)

### 2. Methode

2.1. Herstellen der Testlösungen
   Von jeder Probe wurden am Vortag der Untersuchung Testlösungen von 0,1 % (w/v) in CSL angesetzt. Dazu wurden je 100 ml CSL im Wasserbad auf 60 °C temperiert. Von jeder Probe wurde 0,1 g in jeweils 100 ml CSL von 60 °C eingewogen. Die Ansätze wurden von Hand kräftig geschüttelt und bei 30 °C über Nacht im Brutschrank belassen.
2.2. Herstellen der Testkeimsuspensionen
   Corynebacterium xerosis über 3 bis 4 Tage anzüchten. Übrige Keime in Bouillon bzw. durch Abschwemmen gewinnen.
2.3. Kontamination der Proben und Ermittlung der Keimzahl reduktion
   Von jeder Testlösung wurden für jeden Testkeim 20 ml in sterile 50 ml Braunglasflaschen mit Glasperlen abgefüllt und mit 0,2 ml Keimsuspension kontaminiert. Als Kontrollen wurden je Testkeim 20 ml CSL ohne Probe mitgeführt. Die kontaminierten Proben wurden auf der Schüttelmaschine 3 min geschüttelt und bis zu den Entnahmen bei 30 °C im Brutschrank gehalten.
   Zu den Entnahmezeitpunkten (1, 2, 3, 24 und 48 Stunden) wurde aus jedem Ansatz 1 ml entnommen, in jeweils 9 ml NaCl-Pepton-Pufferlösung mit EG-Enthemmer überführt und die Koloniezahl ermittelt.
   Als 0 Stunden-Werte wurden die Koloniezahlen der verwendeten Testkeimsuspension unter Berücksichtigung der 10⁻²-Verdünnung bei der Probenkontamination angegeben.

### 3. Ergebnisse

Die Einzelergebnisse der Proben sind in folgenden Diagrammen dargestellt. Zusätzlich sind in jedem Diagramm die Keimpopulationen einer wirkstofffreien Blindprobe als Kontrollwert nach 24 Stunden Bebrütung eingetragen.

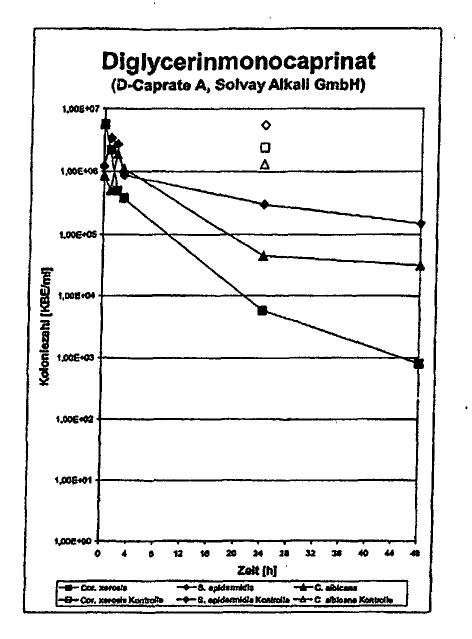

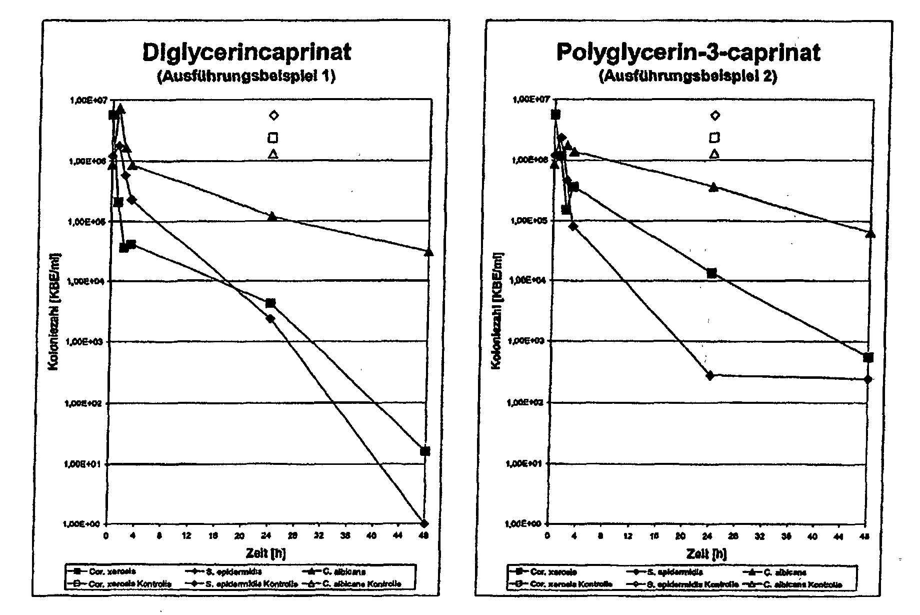

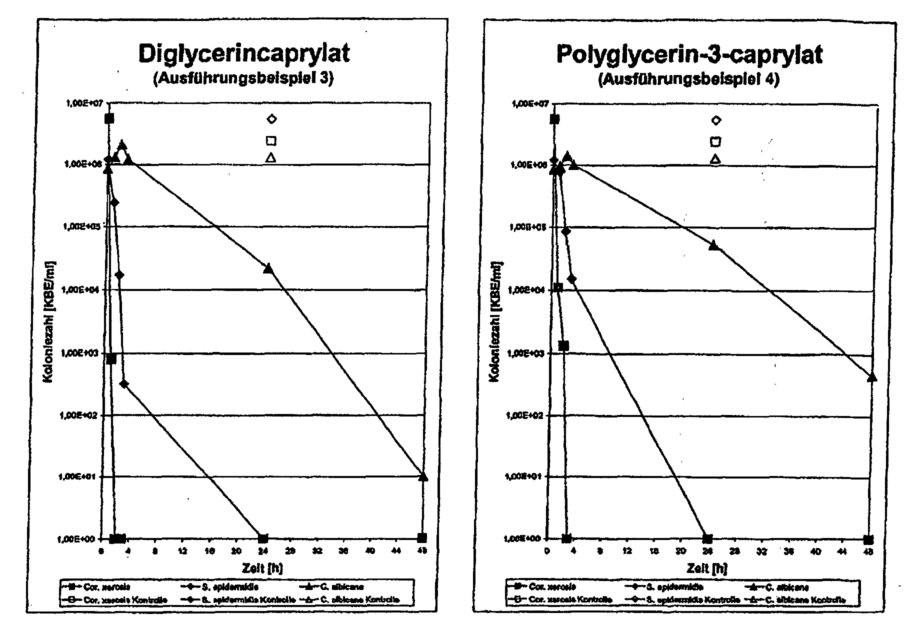

### B) Gegen Malassezia furfur

In gleicher Vorgehensweise wie unter A beschrieben wird die Wirksamkeit von Diglycerincaprylat, wie in Ausführungsbeispiel 3 hergestellt, gegen M. furfur getestet. M. furfur steht in ursächlichem Zusammenhang mit der Bildung von Schuppen.

Diglycerincaprylat wurde in Wasser gelöst, so dass eine Lösung mit 3,0 Gew.-% Gehalt entsteht. Diese Lösung wird mit Keimsuspension versetzt, durch Schütteln homogenisiert und bei 30 °C im Brutschrank gehalten. Eine zweite Lösung ohne Zusatz von Diglycerincaprylat wird als Kontrolle mitgeführt.

Die folgenden Resultate wurden erhalten:

| Probenahme, Zeit (h) | 0 | 1 | 2 | 4 | 24 |
|---|---|---|---|---|---|
| Kontrolle, Keimzahl/ml | 1x20⁵ | n.b. | n.b. | n.b. | 1x10⁴ |
| 0,3 % Diglycerincaprylat, Keimzahl/ml | 1x10⁵ | <10 | <10 | <10 | <10 |
| n.b. = nicht bestimmt | | | | | |

### Beispiel 6: Kosmetische Formulierungen

Es folgen Beispiele für Formulierungen, in denen die erfindungsgemäßen Produkte eingesetzt werden können.

### Formulierung 1: Clear Deodorant Pumpspray

| Phase A: | |
|---|---|
| Produkt aus Beispiel 4 | 0,30 % |
| Trideceth-12 | 2,00 % |
| Dipropylenglykol | 4,00 % |
| Parfüm | 0,90 % |
| | |

| Phase B: | |
|---|---|
| Wasser | ad 100,00 |
| | |
| Konservierungsmittel | q.s. |
| Zitronensäure (50%ig) | q.s. |

Die unter Phase A genannten Bestandteile werden unter Rühren in der angegebenen Reihenfolge zusammengefügt und anschließend langsam mit Wasser (Phase B) aufgefüllt. Der pH-Wert wird mit Zitronensäure auf 5,5 eingestellt.

### Formulierung 2: O/W-Emulsion (sprühbar)

| Phase A: | |
|---|---|
| Glycerinstearat (und) Ceteth-20 | 3,00 % |
| (z.B. TEGINACID® H, Degussa) | |
| Stearylalkohol | 1,00 % |
| Produkt aus Beispiel 4 | 0,30 % |
| Dimethicone | 0,50 % |
| Cetearylethylhexanoat | 4,00 % |
| Capryl/Caprin-Triglycerid | 4,00 % |
| | |

| Phase B: | |
|---|---|
| Glycerin | 3,00 % |
| Wasser | ad 100,00 % |
| Zitronensäure (50 %ig) | pH = 6-7 |
| | |
| Konservierungsmittel | q.s. |
| Parfüm | q.s. |

Die Phasen A und B werden auf 70 bis 75 °C erwärmt. Phase A wird unter Rühren zu Phase B gegeben und anschließend homogenisiert. Unter Rühren wird auf 30 °C abgekühlt.

wichtig: Falls Phase A vorgelegt werden sollte, muss Phase B ohne Rühren zugegeben werden.

### Formulierung 3: Clear Deodorant Roll On

| Phase A: | |
|---|---|
| Produkt aus Beispiel 4 | 0,30 % |
| Trideceth-12 | 2,00 % |
| Dipropylenglykol | 2,00 % |
| Parfüm | 0,50 % |
| PEG-14 Dimethicone | 1,00 % |
| Wasser | ad 65,00 % |
| | |

| Phase B: | |
|---|---|
| Hydroxyethylcellulose (2 % in Wasser) | 35,00 % |
| | |
| Konservierungsmittel | q.s. |
| Zitronensäure (50 %ig) | q.s. |

Die unter Phase A genannten Bestandteile werden unter Rühren in der angegebenen Reihenfolge zusammengefügt. Phase A wird unter Rühren zu Phase B gegeben. Der pH-Wert wird mit Zitronensäure auf 5,5 eingestellt.

Die unter Phase A genannten Bestandteile werden unter Rühren in der angegebenen Reihenfolge zusammengefügt und anschließend langsam mit Wasser (Phase B) aufgefüllt.

### Beispiel 7: Kosmetischer Anwendungstest

Zur Anwendung kommen zwei Formulierungen. Diese sind Formulierung 2 aus Beispiel 6 und als Placebo dieselbe Formulierung, in der das erfindungsgemäße Produkt (aus Beispiel 4) durch nicht verestertes Polyglycerin mit gleicher Verteilung ersetzt worden ist. Der Achselgeruch von 20 Probanden wird vor und nach der Anwendung der Formulierung 2 oder der Placebo-Formulierung durch drei Experten geprüft. Im Einzelnen beinhaltet der Test folgende Schritte:
1. Die Achselhöhle wird mit Seife gewaschen, der Geruch wird durch Experten bewertet,
2. Das Produkt wird einmal in einer Achselhöhle angewendet. Nach 6 und 24 h wird der Geruch geprüft und der Unterschied bewertet.

Das Ergebnis dieser Untersuchung ist, dass sowohl nach 6 als auch nach 24 stündiger Anwendung eine deutliche Verbesserung des Geruches der mit der erfindungsgemäß behandelten Achselhöhle verglichen mit der Placebo behandelten Achselhöhle festgestellt wird.

## Patentansprüche

1. Verfahren zur Bekämpfung von grampositiven Bakterien, gramnegativen Bakterien, Mykobakterien, Dermatophyten, Sprossund Fadenpilzen, Viren und Sporen in kosmetischen Formulierungen unter Verwendung von Mono- und Diestern des Diund/oder Triglycerins mit einem Verhältnis von Mono- zu Diestern im Bereich von 20:80 bis 80:20, erhätlich durch Umsetzung des Di- und/oder Triglycerins in Gegenwart von Lipasen und Esterasen als Ver-/Umesterungskatalysatoren mit geradkettigen oder verzweigten, gegebenenfalls OH-Gruppen und/oder Doppelbindungen enthaltenden Fettsäuren mit 6 bis 14 C-Atomen in der Hauptkette.

2. Verfahren zur Bekämpfung von grampositiven Bakterien, gramnegativen Bakterien, Mykobakterien, Dermatophyten, Sprossund Fadenpilzen, Viren und Sporen in kosmetischen Formulierungen gemäß Anspruch 1 für den Bereich der Körperreinigung und Körperpflege.

3. Verfahren zur Bekämpfung von grampositiven Bakterien, gramnegativen Bakterien, Mykobakterien, Dermatophyten, Sprossund Fadenpilzen, Viren und Sporen in kosmetischen Formulierungen gemäß Anspruch 1 zur Herstellung kosmetischer Formulierungen gegen Körpergeruch und gegen Schuppenbildung.

4. Verfahren zur Bekämpfung von grampositiven Bakterien, gramnegativen Bakterien, Mykobakterien, Dermatophyten, Sprossund Fadenpilzen, Viren und Sporen in kosmetischen Formulierungen gemäß Anspruch 1 zur Herstellung von kosmetischen Formulierungen gegen unreine Haut und leichte Formen der Akne.

## Claims

1. Method for controlling Gram-positive bacteria, Gram-negative bacteria, mycobacteria, dermatophytes, yeast, fungi and hyphal fungi, viruses and spores in cosmetic formulations using mono- and diesters of di- and/or triglycerol in a ratio of mono- to diesters in the range from 20:80 to 80:20, obtainable by reacting the di- and/or triglycerol, in the presence of lipases and esterases as (trans)esterification catalysts, with straight-chain or branched fatty acids having 6 to 14 carbon atoms in the main chain and optionally containing OH groups and/or double bonds.

2. Method for controlling Gram-positive bacteria, Gram-negative bacteria, mycobacteria, dermatophytes, yeast, fungi and hyphal fungi, viruses and spores in cosmetic formulations according to Claim 1 for the field of body cleansing and bodycare.

3. Method for controlling Gram-positive bacteria, Gram-negative bacteria, mycobacteria, dermatophytes, yeast, fungi and hyphal fungi, viruses and spores in cosmetic formulations according to Claim 1 for the preparation of cosmetic formulations against body odor and against formation of dandruff.

4. Method for controlling Gram-positive bacteria, Gram-negative bacteria, mycobacteria, dermatophytes, yeast, fungi and hyphal fungi, viruses and spores in cosmetic formulations according to Claim 1 for the preparation of cosmetic formulations against bad skin and mild forms of acne.

## Revendications

1. Procédé pour la lutte contre des bactéries à Gram positif, des bactéries à Gram négatif, des myco-bactéries, des dermatophytes, des blastomycètes et des champignons filamenteux, des virus et des spores dans des compositions cosmétiques avec utilisation de mono- et diesters du di- et/ou triglycérol à un rapport des monoesters aux diesters dans la plage allant de 20:80 à 80:20, pouvant être obtenus par réaction du di- et/ou triglycérol, en présence de lipases et d'estérases en tant que catalyseurs d'estérification/trans-estérification, avec des acides gras à chaîne droite ou ramifiée, comportant éventuellement des groupes OH et/ou des doubles liaisons, ayant de 6 à 14 atomes de carbone dans la chaîne principale.

2. Procédé pour la lutte contre des bactéries à Gram positif, des bactéries à Gram négatif, des myco-bactéries, des dermatophytes, des blastomycètes et des champignons filamenteux, des virus et des spores dans des compositions cosmétiques selon la revendication 1, pour le domaine du nettoyage du corps et du soin du corps.

3. Procédé pour la lutte contre des bactéries à Gram positif, des bactéries à Gram négatif, des myco-bactéries, des dermatophytes, des blastomycètes et des champignons filamenteux, des virus et des spores dans des compositions cosmétiques selon la revendication 1, pour la fabrication de compositions cosmétiques contre l'odeur corporelle et contre la formation de pellicules.

4. Procédé pour la lutte contre des bactéries à Gram positif, des bactéries à Gram négatif, des myco-bactéries, des dermatophytes, des blastomycètes et des champignons filamenteux, des virus et des spores dans des compositions cosmétiques selon la revendication 1, pour la fabrication de compositions cosmétiques contre la peau malsaine et des formes légères de l'acné.
